# EUROPÄISCHE PATENTSCHRIFT

(11) **EP 0 983 989 B1**
(45) Veröffentlichungstag und Bekanntmachung des Hinweises auf die Patenterteilung: **27.03.2002**
(21) Anmeldenummer: 99116318.9
(22) Anmeldetag: 19.08.1999
(51) Int. Cl.: C07C 49/587, C11B 9/00, A61K 7/46

(54) **Methylcyclotetradec-5-en-1-one**
Methylcyclotetradec-5-ene-1-one
Méthylcyclotétradéc-5-ène-1-one

(30) Priorität: 03.09.1998 CH 180498
(43) Veröffentlichungstag der Anmeldung: 08.03.2000
(73) Patentinhaber: Givaudan SA, 1214 Vernier-Genève (CH)
(72) Erfinder: Helmlinger, Daniel, Dr., 8600 Dübendorf (CH); Frater, Georg, Dr., 8400 Winterthur (CH); Müller, Urs, 8600 Dübendorf (CH)
(74) Vertreter: Patentanwälte Schaad, Balass, Menzl & Partner AG

(56) Entgegenhaltungen:
- EP-A- 0 584 477
- PATENT ABSTRACTS OF JAPAN vol. 004, no. 106 (C-020), 30. Juli 1980 (1980-07-30) & JP 55 066534 A (TAKASAGO CORP), 20. Mai 1980 (1980-05-20)
- DATABASE WPI Section Ch, Week 7930 Derwent Publications Ltd., London, GB; Class D23, AN 1979-55888B XP002117059 & SU 627 118 A (HETEROORG CPDS AS USSR), 17. August 1978 (1978-08-17)

## Beschreibung

Die Erfindung betrifft Methylcyclotetradec-5-en-1-one sowie Riechstoffzusammensetzungen, enthaltend mindestens ein Methylcyclotetradec-5-en-1-on.

Obwohl G. Ohloff, Fortschritte der chemischen Forschung Vol. 12/2, 201 angibt, dass am Zustandekommen des Moschusgeruches macrocyclische Carbonylverbindungen mit mehr als 13, jedoch weniger als 19 Kohlenstoffe beteiligt sind, haben alle kommerziellen Moschusriechstoffe Ringgrössen von 15, 16 oder 17 Kohlenstoffatomen. Über die geruchlichen Eigenschaften von vierzehngliedrigen Ringketonen ist nur in vereinzelten Fällen und äusserst wenig bekannt.

In der US-A 4,183,965 wird die Verwendung eines Gemisches von 2- und 3-Cyclotetradecen-1-on zum Herabsetzen des bitteren Geschmacks von Lebensmitteln beschrieben. Die Verbindungen können auch in der Parfümerie eingesetzt werden und wirken süss, moschusartig exaltoneartig, wachsig, wurzelig.

In der JP-A 55-66534 ist die Synthese von 5-cis-cyclotetradecen-1-on mit moschusartigem Charakter durch Erhitzen von 1-Vinyl-3-cis-cyclododecen-1-ol mit einem Alkalimetall oder Alkalihydrid beschrieben. E. Yoshi and S. Kimoto: Chem. Pharm. Bull 17, 629, 1969 und nachträglich M. Karpf und A. S. Dreiding: Helvetica Chimica Acta, Vol. 58(8), 2409, 1975 beschreiben die Synthese von einem Gemisch von cis- und trans-3-Methylcyclotetradec-2-en-1-one und 3-Methylcyclotetradec-3-en-1-one. Geruchliche Eigenschaften dieser Verbindungen werden nicht angegeben.

Die Herstellung von 3-Methyl-trans-cyclopentadec-5-enon wird von R.W. Thies und K.P. Daruwala: J.Org.Chem. 1987, 52, 3798 durch Behandlung von trans- oder cis-1-(1-Propenyl)-trans-cyclotridec-3-en-1-ol mit Kaliumhydrid in Hexamethylphosphortriamid beschrieben.

In der US-A 5,354,735 werden cis- und trans-Isomere von 3-Methylcyclopentadec-5-en-1-on als Riechstoffbestandteile mit moschusartigen Eigenschaften beschrieben. Das cis-Isomere ist stärker, eleganter und riecht eher nach Moschus und weniger animalisch als das trans-Isomere, welches eher Nitromoschus Charakter und Ambrettesamen Geruch hat.

Es wurde nun überraschenderweise gefunden, dass Verbindungen der Formel I 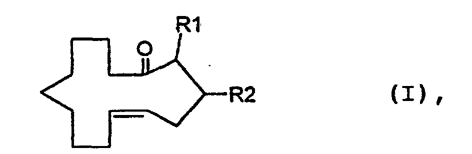 worin R¹ und R² Wasserstoff oder Methyl bedeuten, mit der Bedingung, dass, wenn R¹ für Methyl steht, R² Wasserstoff ist und wenn R¹ für Wasserstoff steht, R² Methyl ist, wobei die Verbindungen der Formel I in der Z- und/oder in der E-Form vorliegen können, einen starken moschusartigen, d.h. nitro moschusartigen, pudrigen, fruchtigen Geruch aufweisen und gleichzeitig einen niedrigen Schwellenwert und eine sehr gute Haftfestigkeit besitzen.

Besonders bevorzugte Verbindung der Formel I ist das E-3-Methylcyclotetradec-5-en-1-on, das überraschenderweise einen mehr als zehnfach stärkeren Geruch als die entsprechende Z-Verbindung, das Z-3-Methylcyclotetradec-5-en-1-on, aufweist. Das E-3-Methylcyclotetradec-5-en-1-on besitzt einen gaschromatographischen Schwellenwert von 0,2 ng währenddem das Z-3-Methylcyclotetradec-5-en-1-on einen solchen von 2,2 ng besitzt.

Die Verbindungen der Formel I lassen sich generell wie die bekannten Moschusriechstoffe einsetzen, sie harmonieren also mit einer Vielzahl natürlicher sowie synthetischer Produkte, die häufig in Riechstoffkompositionen eingesetzt werden. Insbesondere in der Basisnote erzielen sie in Kombination mit amberähnlichen und holzigen Akkorden, wie Patchouliöl, Cedern- und Sandelholzriechstoffen, interessante Effekte. Blumige Herz(Mittel)noten verleihen den Verbindungen der Formel I Eleganz und Strahlungskraft. Einige Beispiele für besonders gut harmonisierende Stoffklassen sind:

Naturprodukte, wie Eichenmoos Absolue, Geraniumöl, Jasmin Absolue, Patchouliöl, Rosenöl, Sandelholzöl,Vetiverol und Ylang-Ylang-Öl.

Alkohole, wie Citronellol, Ebanol®, Geraniol, Linalool, Phenylethylalkohol und Sandalore®.

Aldehyde und Ketone, wie Florozone® (3-(4-Ethylphenyl)2,2-dimethyl-propional), Hydroxycitronellal, Iso-E-Super®, Isoraldein®, Maltol, Methyl-cedrylketon, Methyl-jonon und Vanillin.

Ether und Acetale, wie Ambrox, Geranylmethylether, Rosenoxid und Spirambrene®, (2',2',3,7,7-Pentamethyl-spiro-[bicyclo[4.1.0]heptan-2-5'-[1,3]dioxan].

Ester und Lactone, wie Berryflor®, γ-Decalacton und γ-Undecalacton.

Die Verbindungen I können bei der Herstellung von Kompositionen und - wie obige Aufzählung zeigt - unter Verwendung einer breiten Palette bekannter Riechstoffe bzw. Riechstoffgemische verwendet werden.

Die vorteilhaften Eigenschaften der Verbindungen der Formel I ermöglichen einen breiten und vielseitigen Einsatz einerseits in süssen orientalischen Kreationen, als auch andererseits in den Duftrichtungen "Fougère", "Chypre" und "Floral".

Auf Grund der niedrigen Schwellenwerte und der guten Haftfestigkeit können die Verbindungen der Formel I sowohl in der Luxusparfümerie, in Kompositionen für kosmetische Produkte, als auch für Massenerzeugnisse, wie Detergenzien, eingesetzt werden.

Die Verbindungen der Formel I können in weiten Konzentrationsgrenzen eingesetzt werden, die beispielsweise von 0.1% in Detergenzien bis 40 Gew.% in alkoholischen Lösungen reichen können. Die bevorzugten Konzentrationen liegen zwischen 3 und 20 Gew.%. Konzentrationen ausserhalb der obigen Grenzen sind auch möglich, da der erfahrene Parfümeur auch mit geringeren oder mit höheren Konzentrationen neuartige Wirkungen erzielen kann. Kompositionen, die eine oder mehrere der Verbindungen der Formel I enthalten, können für alle Arten von parfümierten Verbrauchsgütern, wie Eaux de Cologne, Eaux de Toilette, Extraits, Lotionen, Crèmes, Shampoos, Seifen, Salben, Puder, Desodorantien, Detergenzien und andere Haushaltprodukte etc. eingesetzt werden.

Bei der Herstellung von Kompositionen können die Verbindungen der Formel I zusammen mit anderen Riechstoffen bzw. Riechstoffgemischen in bekannter Art, wie beispielsweise von W.A. Poucher, Perfumes, Cosmetics, Soaps, Vol. 2, Aufl. 7 Chapman und Hall, London 1974, beschrieben, eingesetzt werden.

Die Verbindungen der Formel I können nach verschiedenen Methoden hergestellt werden.

Die Verbindung der Formel I, in der R¹ H und R² CH₃ bedeuten, wird nach einer ersten Methode im Gemisch mit einer Verbindung der Formel II 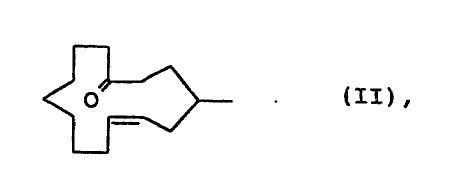 d.h. cis- und trans-4-Methylcyclotetradec-6-en-1-on hergestellt. Dabei unterwirft man (4-Carboxy-3-methyl-butyl)-triphenylphosphoniumbromid einer Wittig-Reaktion, beispielsweise durch Behandlung mit K-t-butylat in Tetrahydrofuran und nachträglicher Zugabe von 9-Oxo-nonansäuremethylester.Das erhaltene Produkt 3-Methyl-tetradec-5-en-disäuredimethylester wird einer Acyloin Kondensation unterworfen und anschliessend mit Acetanhydrid Pyridin behandelt. Das resultierende Gemisch von hauptsächlich Z-4-Methyl-2-oxo-cyclotetradec-6-enylacetat und Z-3-Methyl-14-oxo-cyclotetradec-5-enylacetat wird mit Calcium in Ammoniak bei tiefer Temperatur (-30°C → -70°C) behandelt, wobei der Überschuss von Calcium mit Brombenzol zerstört wird.

Auf diese Weise erhält man ein Gemisch von:
E-3-Methyl-cyclotetradec-5-enon 11%
Z-3-Methyl-cyclotetradec-5-enon 35 %
Z-4-Methyl-cyclotetradec-6-enon 49 %

Geruch: stark moschusartig (nitromoschus), puderartig.

Eine zweite Methode zur Herstellung der Verbindung I (R¹=H, R²=CH₃), bevorzugt in der trans-Form, startet mit 2-Chlorcyclododecanon oder 2-Bromcyclododecanon, die in einer Stufe aus Cyclododecanon gewonnen werden können (JP 491 093 39, EP-A-051 233). Durch Behandlung von 2-Chlorcyclododecanon mit Lithiumcarbonat in N-Methylpyrrolidon bei Rückflusstemperatur erhält man ein Gemisch von:
5 % Z-Cyclododec-2-en-1-on
6 % E-Cyclododec-2-en-1-on
13 % Cyclododecanon
57 % E-Cyclododec-3-en-1-on
16 % Z-Cyclododec-3-en-1-on

Das oben erwähnte Gemisch wird mit 1-Brom-1-propenyl-magnesium-bromid in Tetrahydrofuran behandelt. Man erhält ein Gemisch von hauptsächlich cis/trans-1-(1-Propenyl)-cyclododec-3-en-1-ol. Diese Verbindung ergibt nach Behandlung mit Natriumhydrid in N-Methylpyrrolidon ein Gemisch von E-3-Methylcyclotetradec-5-en-1-on, Z-3-Methylcyclotetradec-5-en-1-on (E:Z = 3:1) und die zwei diastereomeren 3-Methyl-4-vinylcyclododecanone.

Das Gemisch riecht sehr stark moschusartig (nitromoschus), pudrig, linear, nach frischer Wäsche (getrocknet in der Sonne). Der schwache, holzige Geruch der zwei diastereomeren 3-Methyl-4-vinylcyclododecanone wird völlig von den beiden erfindungsgemässen Moschusverbindungen E-3-Methylcyclotetradec-5-en-1-on, Z-3-Methylcyclotetradec-5-en-1-on überdeckt.

Ein ähnliches Gemisch von E- und Z-3-Methylcyclotetradec-5-en-1-on kann erhalten werden durch Pyrolyse des Silylethers von cis/trans Propenyl-cyclododec-3-en-1-ol.

Die Verbindungen der Formel I (R¹=CH₃, R²=H) können wie folgt hergestellt werden:

Ein Gemisch von hauptsächlich E-Cyclododec-3-en-1-on (74%) und Z-Cyclododec-3-en-1-on (19%) wird mit Isopropenylmagesiumbromid in Tetrahydrofuran behandelt, wobei hauptsächlich E-1-Isopropenyl-cyclododec-3-en-1-ol entsteht. Das gereinigte E-1-Isopropenyl-cyclododec-3-en-1-ol wird in N,N-Dimethylacetamid in Gegenwart von 18-Crown-6 mit Kaliumhydrid behandelt, wobei ein Gemisch von diastereomeren 2-Methyl-4-vinylcyclododecanon und E-2-Methylcyclotetradec-5-en-1-on ensteht.

Geruch von E-2-Methylcyclotetradec-5-en-1-on: Moschus (Nitromoschus), pudrig, süss.

Durch die nachfolgenden Beispiele wird die Erfindung weiter Veranschaulicht.

### Beispiel 1

143 g (0,313 Mol) (4-Carboxy-3-methylbutyl) -triphenyl-phosphoniumbromid werden pulverisiert und in 630 ml Tetrahydrofuran vorgelegt. Die erhaltene Suspension wird 10 Minuten gerührt, dann auf -20°C abgekühlt und mit 70 g (0,625 Mol) Kalium-t-butylat schnell in 315 ml Tetrahydrofuran aufgeschlämmt. Die Temperatur steigt auf 0°C und das Reaktionsgemisch färbt sich orange. Es wird 45 Minuten zwischen -5°C und 0°C gerührt. Dann wird auf -20°C abgekühlt und mit 76 g (Reinheit 70%) (0,285 Mol) 9-Oxo-nonansäuremethylester versetzt. Die Temperatur steigt auf -1°C. Man lässt auf Raumtemperatur erwärmen (1 Stunde und 30 Minuten) und rührt eine weitere Stunde bei 40°C. Das Reaktionsgemisch wird auf 1,5 l Eiswasser gegossen, mit 25 ml 30% NaOH auf einen pH-Wert von 14 eingestellt und zweimal mit 700 ml Aether extrahiert. Der pH-Wert der wässrigen Phasen wird mit 100 ml ortho-Phosphorsäure auf 2 - 3 eingestellt und zweimal mit 500 ml Aether extrahiert. Die organische Phase wird mit 300 ml Wasser und 300 ml gesättigter Kochsalzlösung gewaschen, über Na₂SO₄ getrocknet und eingedampft. Das Rohprodukt 102,9 g wird mit 360 ml Methanol verdünnt, 3,4 g p-Toluolsulfonsäure werden dazugegeben und 3 Stunden und 30 Minuten rückflussiert. Dann wird ein Überschuss von NaHCO₃ (fest) zugegeben und eingedampft. Das Rohprodukt (108,9 g) wird über 1 kg Kieselgel 60 Merck (0,040 mm - 0,063 mm) mit 6 l Hexan/Aether 4:1 und 2 l Hexan/Aether 3:1 chromatographiert. 57,3 g Z-3-Methyltetradec-5-endisäuredimethylester werden erhalten.

Spektroskopische Daten des Produktes:
- IR (flüssig):: 3004; 2928; 2855; 1740; 1458; 1436; 1364; 1251; 1198; 1168; 1011.
- NMR:: (CDCl₃, 200 MHz) 5,4 (2H) m; 3,66 (6 H) s.
- MS:: 298 (0,8); 266 (20); 248 (7); 235 (34); 224 (60); 206 (10); 192 (28); 175 (8); 164 (19); 150 (25); 136 (19); 123 (18); 109 (38); 95 (66); 87 (30); 81 (94); 75 (69); 68 (69); 59 (75); 55 (100); 41 (88); 29 (27).

### Beispiel 2

In einer trocknen Apparatur mit Ölbad werden 2 l Xylol vorgelegt und während 30 Minuten wird Argon durchgeleitet. Das Ölbad wird auf 148°C erhitzt. Ab 100°C Innentemperatur werden 17,2 g (0,750 Mol) Natrium portionenweise zugegeben. Danach wird während 4 Stunden eine Lösung von 56 g (0,188 Mol) Z-3-Methyl-tetradec-5-endisäuredimethylester in 150 ml Xylol zugetropft.

Die Innentemperatur steigt bis 134°C. Dann wird 30 Minuten bei dieser Temperatur gerührt, auf Raumtemperatur abgekühlt und 120 ml EtOH und 50 ml Wasser werden tropfenweise zugegeben. Die organische Phase wird mit 400 ml Wasser und 200 ml gesättigter Kochsalzlösung gewaschen, über Na₂SO₄ getrocknet und eingedampft. Auf diese Weise werden 36,6 g Rohprodukt erhalten. Dieses Rohprodukt wird in 75 ml Pyridin gelöst, 15,4 g Essigsäureanhydrid werden zugegeben und während 4 Stunden bei 80°C gerührt. Das Produkt wird auf 400 ml Eiswasser gegossen, mit konzentrierter Salzsäure auf pH 2 eingestellt und zweimal mit 200 ml Aether extrahiert. Die organischen Phasen werden mit Wasser und gesättigter Kochsalzlösung gewaschen und über Natriumsulfat getrocknet und eingedampft.

Man erhält auf diese Weise 41,5 g Rohprodukt, das über 1 kg Kieselgel 60 Merck (0,040 mm - 0,063 mm) chromatographiert wird. 30,9 g eines Gemisches von hauptsächlich Z-4-Methyl-2-oxo-cyclotetradec-6-enylacetat und Z-3-Methyl-14-oxo-cyclotetradec-5-enylacetat werden erhalten.

Spektroskopische Daten des Gemisches:
- IR (flüssig):: 3006; 2928; 2857; 1746; 1727; 1461; 1373; 1236; 1087; 1026.
- NMR:: (CDCl₃, 200 MHz) 5,45 (2H) m; 5,1 (2H) m; 2,15 (3H) s.
- MS:: 280 (1); 238 (9); 220 (9); 202 (3); 191 (1); 177 (4); 163 (4); 149 (5); 135 (7); 121 (12); 111 (16); 95 (20); 81 (28); 67 (18); 55 (28); 43 (100); 29 (8).

### Beispiel 3

35,8 g (0,893 Mol) Calcium werden innerhalb von 20 Minuten bei -50°C 1,4 l Ammoniak zugegeben und 20 Minuten wird bei -60°C gerührt. Innerhalb von 2 Stunden werden bei -50°C 30 g eines Gemisches von hauptsächlich Z-4-Methyl-2-oxo-cyclotetradec-6-enylacetat und Z-3-Methyl-14-oxo-cyclotetradec-5-enylacetat in 330 ml Tetrahydrofuran tropfenweise zugegeben und während 15 Minuten bei -70°C gerührt. Dann werden 160 ml Brombenzol innerhalb von 30 Minuten tropfenweise zugegeben. Das Gemisch wird auf Raumtemperatur erwärmen gelassen und das Ammoniak wird abgedampft. Dann wird das Produkt auf Eis gegossen, auf pH 3 mit Orthophosphorsäure angesäuert und mit Aether extrahiert. Die organische Phase wird mit Wasser, gesättigter Kochsalzlösung, gewaschen und über Na₂SO₄ getrocknet und eingedampft. Das Rohprodukt (39,8 g) wird über 800 g Kieselgel 60 Merck (0,040 - 0,063 mm) chromatographiert. Man erhält auf diese Weise 13,7 g eines Gemisches von:
E-3-Methyl-cyclotetradec-5-enon 11 %
Z-3-Methyl-cyclotetradec-5-enon 35 %
Z-4-Methyl-cyclotetradec-6-enon 49 %

Geruch des Gemisches: Moschus (nitromoschus), pudrig.

Geruch von E-3-Methyl-cyclotetradec-5-enon: Moschus, pudrig, animalisch, ambraartig.

Die einzelnen Verbindungen konnten durch zusätzliche Chromatographie über Kieselgel (0,040 - 0,063 mm) mit 10% Silbernitrat rein gewonnen werden.

Spektroskopische Daten von: Z-3-Methyl-cyclotetradec-5-en-1-on:
- IR: (flüssig):: 3008; 2929; 2859; 1710; 1460; 1408; 1369; 1047; 718.
- ¹H-NMR:: (CDCl₃ 200 MHz) 5,46 (2H) m; 1,0 (3H) d; J = 7,5 Hz.
- ¹³C-NMR:: (CDCl₃) 211,2 (s); 131,6 (d); 126,7 (d); 49,6 (t); 40,2; (t) 32,8 (t); 30,6 (d) ; 27,3(t); 26,1 (t); 25,6 (t); 25,0 (t) ; 24,7 (t); 21,1 (t); 20,1 (q).
- MS:: 222 (25); 207 (5); 193 (4); 179 (11); 164 (17); 147 (8); 135 (17); 121 (19); 109 (33); 95 (58); 81 (100); 68 (94); 55 (89); 41 (94); 29 (25).

Spektroskopische Daten von Z-4-Methyl-cyclotetradec-6-en-1-on:
- IR (flüssig) :: 3007; 2928; 2858; 1711; 1461; 1408; 1375; 1287; 1124; 1046; 712.
- ¹H-NMR:: (CDCl₃, 200 MHz) 5,35 (1H) m; 5,48 (1H) m; 0,96 (2H) d; J = 7,5 Hz.
- ¹³C-NMR:: (CDCl₃) 212.1 (s); 130,8 (d); 127,7 (d); 40,2 (t); 39,7 (t); 33,4 (d); 33,4 (t) ; 30,8 (t); 27,0 (t); 26,7 (t); 26,1 (t); 25,5 (t); 25,2 (t); 23,2 (t); 19,6 (q).
- MS:: 222 (33); 204 (4); 193 (3); 179 (15); 165 (14); 147 (10); 135 (12); 125 (21) ; 111 (47); 98 (56); 81 (60); 67 (55); 55 (100); 41 (70); 29 (23).

Spektroskopische Daten von E-3-Methyl-cyclotetradec-5-en-1-on:
- IR (flüssig):: 2928; 2856; 1708; 1458; 1441; 1365; 970.
- H-NMR:: (CDCl₃ 400 MHz) 5,32 (2H) m; 2,86 (1H) dd, J = 17 Hz, J = 2 Hz; 2,33 (1H) m; 0,95 (3H) d; J = 6,6 Hz.
- ¹³C-NMR:: (CDCl₃) 212,4 (s); 132, 8 (d); 129,9 (d) ; 46,2 (t); 42,9 (t); 31,1 (t); 29,0 (d) ; 27,2 (t); 26,5 (t); 25,2 (t); 24,8 (t); 24,5 (t); 23,3 (t); 21,1 (q) .
- MS:: 222 (69); 207 (14); 193 (6); 179 (15); 164 (34); 154 (1); 147 (12); 135 (30); 123 (26); 109 (41); 95 (69); 81 (100); 67 (76); 55 (63); 41 (57); 28 (16).

### Beispiel 4

Zu 1,2 l N-Methylpyrrolidon werden 100 g Lithiumcarbonat (1,35 Mol, pulverisiert) und 260 g (1,2 Mol) 2-Chlorcyclododecanon zugegeben. Unter Rühren wird auf 180° - 185°C erhitzt, wobei CO₂ abgespalten wird (Dauer 3 Stunden). Das Rohgemisch wird abgekühlt, auf 2,5 l Wasser gegossen und dreimal mit Hexan ausgeschüttelt, die organische Phase wird dreimal mit Wasser gewaschen, getrocknet und eingedampft. Das Rohprodukt (238 g) wird unter Hochvakuum (0,1 mm) destilliert. Nach einem Vorlauf (4,9 g) werden 105 g eines Produktes mit einem Siedepunkt von 89 bis 95°C/0,1 mm erhalten. Die gaschromatographische Analyse zeigt:
5 % Z-cyclododec-2-en-1-on
6 % E-Cyclododec-2-en-1-on
13 % Cyclododecanon
57 % E-Cyclododec-3-en-1-on
16 % Z-Cyclododec-3-en-1-on

Spektroskopische Daten von Z-Cyclododec-3-en-1-on:
- ¹³C-NMR:: (CDCl₃) 211,0 (s); 132,0 (d); 122,9 (d) ; 43,4 (t); 37,7 (t); 26,6 (t); 26,3 (t); 24,3 (t); 24,1 (t); 23,5 (t); 23,0 (t); 22,6 (t).
- MS:: 180 (62); 162 (6); 151 (19); 137 (25); 123 (25); 111 (52); 98 (100); 81 (68); 67 (82); 54 (84); 41 (60); 27 (12).

Spektroskopische Daten von E-Cyclododec-3-en-1-on:
- H-NMR:: (CDCl₃ 400 MHz) 5,7 - 5,61 d,t,t (1H) ; J = 15,3 Hz; J = 1,2 Hz; J = 7,4 Hz; 5,34 - 5,43 d,t,t (1H); J = 15,2 Hz; J = 1,2 Hz; J= 7,6 Hz; 3.04 (2H) d; J = 7,6 Hz; 2,48 (2H) t; J = 6,8 Hz; 2,03 (2H) m.
- ¹³C-NMR:: (CDCl₃) 209,7 (s); 136,3 (d) ; 122,8 (d) ; 48,3 (t); 39,6 (t); 32,2 (t); 26,3 (t) ; 25,4 (t); 24,9 (t); 24,1 (t); 23,9 (t) ; 22,1 (t).
- MS:: 180 (68); 162 (62); 151 (17); 137 (23); 123 (23); 111 (51); 98 (100); 81 (63); 67 (80); 54 (86); 41 (61); 27 (12).

### Beispiel 5

8,26 g Magnesium (0,34 Mol) werden mit einigen Kristallen Jod aktiviert, 25 ml Tetrahydrofuran werden zugegeben und 41 g (0,34 Mol) 1-Brom-1-propen (cis-trans-Gemisch) gelöst in 120 ml Tetrahydrofuran werden langsam bei 70°C zugetropft (Zutropfzeit 1 Stunde und 30 Minuten). Während 3 Stunden wird bei dieser Temperatur gerührt. Dann wird auf 0°C abgekühlt und 51,2 g eines Gemisches von hauptsächlich E-Cyclododec-3-en-1-on (62 %), Z-Cyclohex-3-en-1-on (18%) und E-Cyclododec-2-en-1-on (7%), hergestellt nach Beispiel 4, gelöst in 100 ml Tetrahydrofuran werden innerhalb von 30 Minuten zugetropft. Anschliessend wird während 1 Stunde und 30 Minuten bei Raumtemperatur gerührt, mit im Eis gekühlter gesättigter Ammoniumchlorid Lösung und Wasser versetzt, 100 ml Aether und 37 g Phosphorsäure zugegeben. Die Phasen werden getrennt und zweimal mit Aether extrahiert. Die organische Phase wird mit gesättigter Kochsalzlösung gewaschen, getrocknet und eingedampft. 60,7 g (96 %) Rohprodukt werden erhalten, das ein Gemisch von hauptsächlich 1-cis/1-trans-Propenylcyclododec-3-en-1-ol ist. Zur Charakterisierung der gebildeten Verbindungen wurden 3,6 g des Rohproduktes über 110 g Kieselgel 60 Merck (0,04 - 0,063 mm) chromatographiert (Elution Hexan/Aether zuerst 9:1, dann 2:1).

Spektroskopische Daten von (1E,3E)-1-(1-Propenyl-cyclo-dodec-3-en-1-ol:
- H-NMR:: (CDCl₃ 400 MHz) 5,71 - 5,51 (3H) m; 5,51 - 5,4 (1H) m; System ABX: 2,33 (1H) dd, J= 7,6; J = 14 Hz, 2,25 (1H) dd J = 6 Hz, J = 14 Hz; 2,1 (2H) dd; J = 5,6; J = 5,6; 1,7 (3H) d, J = 5 Hz.
- ¹³C-NMR:: (CDCl₃) 138,1 (d); 134,7 (d) ; 125,3 (d); 122,8 (d); 74,7 (s); 43,0 (t); 37,1 (t); 33,3 (t) ; 28,6 (t); 27,0 (t); 26,0 (t); 25,5 (t); 24,3 (t); 19,0 (t); 17,8 (q).
- MS:: 222 (2); 207 (17); 204 (64); 189 (7); 179 (9); 175 (10) ; 161 (12) 147 (16) ; 133 (26); 119 (33); 105 (51); 97 (60); 91 (58); 84 (69); 79 (55); 69 (100); 55 (29); 41 (35); 29 (6).

Spektroskopische Daten von (1-Z,3E)-1-(1-Propenyl)-cyclo-dodec-3-en-1-ol:
- H-NMR:: (CDCl₃, 400 MHz) 5,69 - 5,3 (4H) m, 2,4 (2H) d, J = 6,5 Hz, 2,11 (2H) dd J = 6 Hz, J = 6 Hz, 1,9 (3H) d J = 5,5 Hz.
- ¹³C-NMR:: (CDCl₃) 136,2 (d); 134,9 (d); 126,5 (d); 125,3 (d); 75,8 (s); 44,6 (t); 37,8 (t); 33,3 (t) ; 28,5 (t); 27,1 (t); 26,0 (t); 25,1 (t); 24,3 (t); 19,1 (t); 14,5 (q).
- MS:: 222 (1); 208 (14); 204 (27); 189 (3); 179 (7); 175 (6); 166 (5); 161 (6); 151 (9); 147 (8); 133 (12); 124 (12); 119 (16); 105 (25); 97 (44); 91 (28); 84 (51); 79 (29); 69 (100); 55 (20); 41 (25); 29 (4).

### Beispiel 6

In einer getrockneten Apparatur werden 11,1 g Propenyl-cyclododec-3-en-1-ol, hergestellt nach Beispiel 5, (Rohprodukt) in 150 ml N-Methylpyrrolidon vorgelegt und mit 4,8 g Natriumhydrid (55%) versetzt. Eine mässige Wasserstoffentwicklung setzt ein, die Temperatur steigt auf 30°C. Dann wird während 5 Stunden bei 85°C gerührt, abgekühlt und 20 ml Wasser tropfenweise zugegeben. Das Produkt wird auf 200 ml Eiswasser gegossen, mit 10 ml Phosphorsäure auf pH 5 eingestellt und mit Aether extrahiert. Die organische Phase wird mit Wasser und gesättigter Kochsalzlösung gewaschen, getrocknet und eingedampft. Das Produkt (13,6 g) wurde chromatographiert (380 g Kieselgel 0,04 - 0,063 mm: Elution: Hexane/Ether 19:1 und 8:1). Man erhält 3,6 g Produkt neben 2,7 g Ausangsmaterial. Das Produkt besteht aus einem Gemisch von hauptsächlich E-3-Methylcyclotetradec-5-en-1-on, Z-3-Methylcyclotetradec-5-en-1-on (E:Z = 3:1) und erythro und threo 3-Methyl-4-vinylcyclododecanon. Das Gemisch riecht sehr stark moschusartig (nitromoschus), pudrig, linear, nach frischer Wäsche (getrocknet in der Sonne). Die einzelnen Verbindungen konnten durch zusätzliche Chromatographie über 10% NO₃Ag Kieselgel gereinigt werden.

Spektroskopische Daten von 3-Methyl-4-vinylcyclododecanon (1. Diastereomer):
- IR (flüssig):: 3074; 2932; 2866; 1707; 1637; 1467; 1445; 1368; 1323; 1173; 1037; 994; 911.
- H-NMR:: (CDCl₃, 200 MHz 5,7 (1H) m; 5,08 (1H) m; 5,0 (1H) m; 2,96 (1H) dd; J = 18 Hz; J = 11 Hz; 0,84 (3H) d; J = 7,5 Hz.
- ¹³C-NMR:: (CDCl₃) 211,6 (s); 141,3 (d); 114,8 (t) ; 44,4 (t); 43,4 (t); 41,1 (d); 31,8 (d) ; 26,0 (t); 24,4 (t); 23,5 (t); 23,0 (t) ; 22,7 (t); 22,5 (t); 14,9 (q).
- MS:: 222 (1); 207 (2); 193 (5); 165 (4); 151 (8); 37 (14); 123 (17); 109 (29); 95 (42); 81 (60); 67 (90); 55 (98); 41 (100); 29 (27).

Spektroskopische Daten von 3-Methyl-4-vinylcyclododecanon (2. Diastereomere):
- H-NMR:: (CDCl₃, 400 MHz; 5,55 (1H) m; 5,08 (1H) m; 5,05 (1H) m; 0,9 (3H) d; J = 7Hz.
- ¹³C-NMR:: (CDCl₃) 211,7 (s); 138,6 (d); 116,4 (t) ; 49,6 (t); 43,10 (d); 38,2 (t); 32,1 (d) ; 30,7 (t); 26,0 (t); 23,87 (t); 23,85 (t) ; 23,5 (t); 22,8 (t); 21,7 (t); 16,0 (q).
- MS:: 222 (15); 207 (22); 193 (28); 180 (6); 179 (24); 175 (27); 165 (25); 151 (31); 137 (54); 123 (58); 109 (70); 95 (76); 81 (84); 67 (95); 55 (100); 41 (81); 29 (15).

### Beispiel 7

7 g (0.3 Mol) Magnesium werden mit wenig Tetrahydrofuran (50 ml) überschichtet und 3 g 2-Brompropen in 28 ml Tetrahydrofuran werden zugeben. Das Gemisch wird kurz erwärmt, wobei die Reaktion anspringt. Dann werden 27 g 2-Brompropen gelöst in 160 ml Tetrahydrofuran innerhalb von 90 Minuten zugetropf. Dabei wird mit einem Eisbad gekühlt, so dass die Temperatur zwischen 55°C und 60°C bleibt. Dann wird während 20 Minuten rückflussiert, abgekühlt (20°C) und 40 g (0.222 mol) eines Gemisches von hauptsächlich E-Cyclododec-3-en-1-on (74%) und Z-Cyclododec-3-en-1-on (19%) gelöst in 50 ml Tetrahydrofuran werden innerhalb von 1 Stunde zugetropft. Die Temperatur darf 35°C nicht überschreiten. Anschliessend wird 20 Minuten bei Raumtemperatur gerührt. Das Reaktionsprodukt wird auf ein Gemisch von Eis/Wasser und Ammoniumchlorid gegossen, mit Methyl-t-butylether extrahiert, mit Wasser, gesättigter Kochsalzlösung neutral gewaschen, über Magnesiumsulfat getrocknet und eingedampft. Das Rohprodukt (48 g) wurde chromatographiert (Hexan,Methyl-t-butylether 9:1). Auf diese Weise werden 37 g E-1-Isopropenyl-cyclododec-3-en-1-ol erhalten. Nach Umkristallisation wird ein Produkt (33 g ) mit einer Reinheit von 95% gewonnen:

Spektroskopische Daten von E-1-Isopropenyl-cyclododec-3-en-1-ol:
- IR (KBr):: 3285; 2988; 2932; 2861; 1641; 1448; 1396; 1368; 1212; 1024; 1003; 981; 898; 700.
- H-NMR:: (CDCl₃, 400 MHz); 5.43-5.73 (2H) m ;4.8-5 (2H) m; 1.8 (3H) m.
- ¹³C-NMR:: (CDCl₃) 150.22 (s); 134.67 (d) ; 125.71 (d) ; 110.30 (t);76.66 (s);41.27 (t); 34.64 (t); 33.24 (t); 28.42 (t); 26.98 (t); 26.00 (t); 24.93 (t); 24.42 (t); 19.22 (t); 18.81 (q).

### Beispiel 8

Zu 25 g (0.125 Mol) Kaliumhydrid (20% in Öl) werden 200 ml N,N-Dimethylacetamid, 34 g 18-Crown-6 (Fluka) zugeben. Dann werden 18 g (0.085 Mol) E-1-Isopropenyl-cyclododec-3-en-1-ol (Reinheit 95%) zugegeben und schnell auf 120°C erwärmt, wobei eine orangerote Lösung ensteht, die 1 Minute bei 120°C gehalten und dann auf Raumtemperatur abkühlen gelassen wird. Das Reaktionsgemisch wird auf Wasser,Eis und Citronensäure gegossen und mit Hexan extrahiert. Die organische Phase wird mit Wasser, gesättigter Kochsalzlösung neutral gewaschen, über Magnesiumsulfat getrocknet und eingeengt. Das Rohprodukt (20 g) wird mit Hexan und Methyl-t-butylether chromatographiert. Man erhält 4.2 g eines Gemisches von diastereomeren Cyclododecanon, 6.2 g eines Gemisches von diastereomeren 2-Methyl-4-vinyl-cyclododecanon, E-2-Methylcyclotetradec-5-en-1-on und 3 g reines E-2-Methylcyclotetradec-5-en-1-on.

Spektroskopische Daten des Gemisches von diastereomeren 2-Methyl-4-vinyl-cyclododecanon:
- IR (flüssig):: 3075; 2931;.2864; 1705; 1639,1465; 1444; 1360; 912.
- H-NMR:: (CDCl₃, 200 MHz) 5.25-5.65 (1H)m; 5.1-4.9 (2H)m; 1.11 (3H) d; J = 6.5Hz.
- MS:: 222 (14); 207 (7); 193 (24);180 (8); 179 (17); 175 (19); 175 (22); 150 (75); 137 (27);123 (53); 109 (77); 95 (83); 81 (100); 67 (90); 55 (99); 41 (80); 29 (15).

Spektroskopische Daten von E-2-Methylcyclotetradec-5-en-1-on:
- IR (flüssig):: 2929; 2854; 1704; 1458; 1374; 972.
- H-NMR:: (CDCl₃, 200 MHz); 5.35 (2H)m; 1.05 (3H)d; J = 6.5 Hz.
- ¹³C-NMR:: (CDCl₃) 215.75(s); 132.05(d); 130.34(d); 41.08 (t); 40.58 (d); 31.41 (t); 30.81 (t) ; 29.38 (t); 26.88 (t); 26.33 (t); 25.93 (t) ; 24.63 (t); 24.61 (t); 22.92 (t); 15.14 (q) .
- MS:: 222 (100); 207 (4); 193 (41); 175 (17); 165 (31); 150 (46); 140 (34); 135 (24); 121 (40); 109 (61); 95 (77); 81 (85); 67 (94); 55 (89); 41 (77); 99 (15).

| **Weichmacher Akkord (fabric softener accord)** | |
|---|---|
| | Gewichtsanteil |
| E-3-Methyl-cyclotetradec-5-en-1-on (Beispiel 3) | 30 |
| Phenylethylacetat | 30 |
| Benzylalkohol extra | 100 |
| Hexylcinnamylaldehyd | 150 |
| Citronellol extra | 50 |
| Coumarin | 20 |
| Dynascone 10 | 1 |
| Floralozon | 4 |
| Isoraldein 70 | 100 |
| Lilial | 250 |
| Linalool synt. | 100 |
| Methylacetophenon | 5 |
| Methylcedrylketon | 50 |
| Radjanol | 10 |
| Amylsalicylate | 50 |
| Terpineol | 50 |
| | 1000 |

In diesem blumigen, holzigen Akkord für ein fabric softener bringt die Verbindung, hergestellt nach Beispiel 3, Volumen und Sauberkeit. Infolge der guten Substantivität bleiben Frische und Sauberkeit auch auf der trockenen Wäsche erhalten.

| **Parfumerie-Akkord für alkoholisches Parfum (fine fragrance accord)** | |
|---|---|
| | Gewichtsanteil |
| E-3-Methyl-cyclotetradec-5-en-1-on (Beispiel 3 | 50 |
| Benzylacetat | 15 |
| Ethylacetoacetat | 25 |
| Ethylphenylalkohol | 60 |
| Hexylcinnamylaldehyd | 70 |
| Ambrettolid | 10 |
| Ambrofix | 2 |
| Ethylenbrassylat | 100 |
| Citronellol extra | 50 |
| Cyclogalbonat | 4 |
| Cyclohexal | 20 |
| Ethyllinalool | 80 |
| Oxyoctalinformiat | 15 |
| Gardenol | 2 |
| Givescon | 15 |
| Hedion | 300 |
| Indol 10 % PE | 2 |
| Isoraldein 95 | 35 |
| Jasmon cis | 3 |
| Lilial | 80 |
| Methyl Pamplemousse | 20 |
| Schwarzer Pfeffer Ess. | 10 |
| Tricyclal | 2 |
| Tropional | 30 |
| | 1000 |

In diesem blumigen, transparenten Akkord für alkoholische Parfums bringt die Verbindung, hergestellt nach Beispiel 3, Reichtum, ein moschusartigen pudrigen Effekt der sich harmonisch verbindet mit dem blumigen, fruchtigen Teil des Akkords.

## Patentansprüche

1. Verbindungen der Formel I 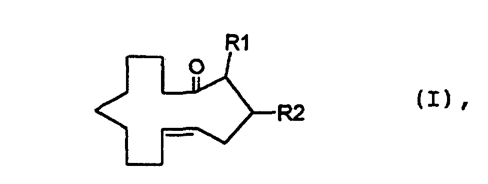 worin R¹ und R² Wasserstoff oder Methyl bedeuten, mit der Bedingung, dass, wenn R¹ für Methyl steht, R² Wasserstoff ist und wenn R¹ für Wasserstoff steht, R² Methyl ist, wobei die Verbindungen der Formel I in der Z- und/oder in der E-Form vorliegen können.

2. E-3-Methylcyclotetradec-5-en-1-on.

3. Z-3-Methylcyclotetradec-5-en-1-on.

4. R-E-3-Methylcyclotetradec-5-en-1-on.

5. S-E-3-Methylcyclotetradec-5-en-1-on.

6. R-Z-3-Methylcyclotetradec-5-en-1-on.

7. S-Z-3-Methylcyclotetradec-5-en-1-on.

8. E-2-Methylcyclotetradec-5-en-1-on.

9. Z-2-Methylcyclotetradec-5-en-1-on.

10. Riechstoffzusammensetzung, enthaltend mindestens eine Verbindung nach einem der Ansprüche 1 bis 9.

## Claims

1. Compounds of the formula I 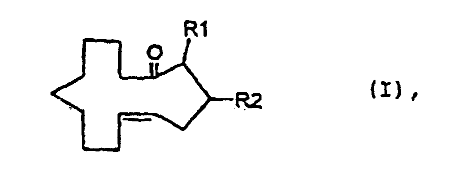 wherein R¹ and R² denote hydrogen or methyl, with the proviso that when R¹ denotes methyl then R² denotes hydrogen, and when R¹ denotes hydrogen R² denotes methyl, and wherein the compounds of the formula I may be present in the Z-form and/or in the E-form.

2. E-3-methylcyclotetradec-5-en-1-one.

3. Z-3-methylcyclotetradec-5-en-1-one.

4. R-E-3-methylcyclotetradec-5-en-1-one.

5. S-E-3-methylcyclotetradec-5-en-1-one.

6. R-Z-3-methylcyclotetradec-5-en-1-one.

7. S-Z-3-methylcyclotetradec-5-en-1-one.

8. E-2-methylcyclotetradec-5-en-1-one.

9. Z-2-methylcyclotetradec-5-en-1-one.

10. odoriferous substance composition containing at least one compound according to claims 1 to 9.

## Revendications

1. Composés de la formule I: 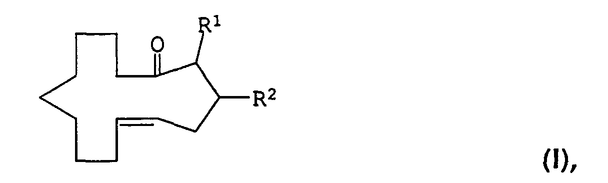 dans laquelle R¹ et R² sont hydrogène ou méthyle, à la condition que, lorsque R¹ est méthyle, R² est hydrogène et, lorsque R¹ est hydrogène, R² est méthyle, les composés de la formule I pouvant être présents en configuration Z et/ou en configuration E.

2. E-3-méthylcyclotétradéc-5-én-1-one.

3. Z-3-méthylcyclotétradéc-5-én-1-one.

4. R-E-3-méthylcyclotétradéc-5-én-1-one.

5. S-E-3-méthylcyclotétradéc-5-én-1-one.

6. R-Z-3-méthylcyclotétradéc-5-én-1-one.

7. S-Z-3-méthylcyclotétradéc-5-én-1-one.

8. E-2-méthylcyclotétradéc-5-én-1-one.

9. Z-2-méthylcyclotétradéc-5-én-1-one.

10. Composition de substance odorante contenant au moins un composé selon l'une des revendications 1 à 9.
